(19) Europäisches Patentamt
European Patent Office
Office européen des brevets

(11) EP 3 711 752 B1

(12) **EUROPEAN PATENT SPECIFICATION**

(45) Date of publication and mention of the grant of the patent:
**20.07.2022 Bulletin 2022/29**

(21) Application number: **17935280.2**

(22) Date of filing: **20.12.2017**

(51) International Patent Classification (IPC):
***A61K 8/34*** (2006.01) ***A61K 8/97*** (2017.01)
***A61K 31/05*** (2006.01) ***A61K 36/185*** (2006.01)
***A61Q 19/02*** (2006.01) ***A61P 17/00*** (2006.01)

(52) Cooperative Patent Classification (CPC):
**A61K 8/9789; A61K 8/347; A61K 31/05; A61K 36/185; A61P 17/00; A61Q 19/02;**
A61K 2800/782

(86) International application number:
**PCT/CN2017/117494**

(87) International publication number:
**WO 2019/119298 (27.06.2019 Gazette 2019/26)**

(54) **USE OF CANNABIDIOL OR CANNABIS EXTRACT IN A COSMETIC METHOD OF SKIN WHITENING**

VERWENDUNG VON CANNABIDIOL ODER CANNABISEXTRAKT IN EINEM KOSMETISCHEN VERFAHREN ZUR HAUTBLEICHUNG

UTILISATION DU CANNABIDIOL OU D'UN EXTRAIT DE CANNABIS DANS UN PROCÉDÉ COSMÉTIQUE DE BLANCHIMENT DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.09.2020 Bulletin 2020/39**

(73) Proprietor: **Hanyi Bio-Technology Company Ltd.**
**Beijing 100020 (CN)**

(72) Inventors:
- **ZHANG, Ke**
  **Beijing 100020 (CN)**
- **TAN, Xin**
  **Beijing 100020 (CN)**
- **YU, Zhaohui**
  **Beijing 100020 (CN)**
- **CHEN, Zheng**
  **Beijing 100020 (CN)**
- **WU, Yanan**
  **Beijing 100020 (CN)**
- **LI, Meng**
  **Beijing 100020 (CN)**

(74) Representative: **Murgitroyd & Company**
**Murgitroyd House**
**165-169 Scotland Street**
**Glasgow G5 8PL (GB)**

(56) References cited:
**CN-A- 106 074 493** **CN-A- 107 308 440**
**CN-A- 107 308 440** **US-A1- 2016 000 843**
**US-A1- 2016 374 911**

- **HWANG YOUNG SUN ET AL: "Cannabidiol upregulates melanogenesis through CB1 dependent pathway by activating p38 MAPK and p42/44 MAPK", CHEMICO-BIOLOGICAL INTERACTIONS, ELSEVIER SCIENCE IRLAND, IR, vol. 273, 7 June 2017 (2017-06-07), pages 107-114, XP085131452, ISSN: 0009-2797, DOI: 10.1016/J.CBI.2017.06.005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

Field

The present invention belongs to the field of cosmetics and relates to use of cannabidiol or cannabis extract in a cosmetic method of skin whitening.

**Background**

Melanocyte is one of the important cells which constitute skin, and is of a dendrite shape. Melanocyte forms skin color by synthesizing melanin. The intensity of skin color is determined by the amount of melanin synthesized by melanocyte. Melanin is a polymeric biological pigment, and is primarily composed of two kinds of quinoid polymers: eumelanin and pheomelanin, wherein, eumelanin is the primary pigment.

From a biochemical point of view, tyrosine is the main raw material for producing melanin; tyrosinase (EC1.4.18.1) is a primary rate-limiting enzyme for transformation of tyrosine into melanin, the activity of the enzyme determines a formation amount of melanin. Tyrosine, under the action of a tyrosinase containing high-valence copper ion ($Cu^{2+}$), and is oxidized to generate 3,4-dihydroxyphenylalanine (DOPA) which is oxidized into dopaquinone by tyrosinase, and further oxidized into 5,6-dihydroxyindole, after polymerization, eumelanin is generated. In melanin synthesis, dopaquinone can also generate pheomelanin via other ways.

Because tyrosinase is the primary rate-limiting enzyme in the course of melanin formation, in order to achieve an ideal skin whitening effect, inhibition against tyrosinase becomes particularly critical.

CN107308440A discloses a stretch mark removing, scar repairing, wrinkle removing and whitening skin care product which comprises a oligopeptides and several plant extracts.

US2016000843 discloses a cannabis cultivar that produces high concentrations of cannabidiol.

HWANG YOUNG SUN ET AL, ("Cannabidiol upregulates melanogenesis through CB1 dependent pathway by activating p38 MAPK and p42/44 MAPK", CHEMICO-BIOLOGICAL INTERACTIONS, vol. 273, 2017, pages 107 - 114) investigate the effects of cannabidiol on melanogenesis and its mechanisms of action in human epidermal melanocytes.

US20160374911A1 discloses inhibitors of tyrosinase for reducing melanin production and provide skin whitening.

Cannabidiol (CBD) is one of cannabinoids, it is generally extracted from natural plant cannabis. Cannabidiol has no psychiatric effect, and has therapeutic actions on anxiety, depression, convulsion, tumor, etc. Cannabidiol has a structural formula as shown by the following Formula I:

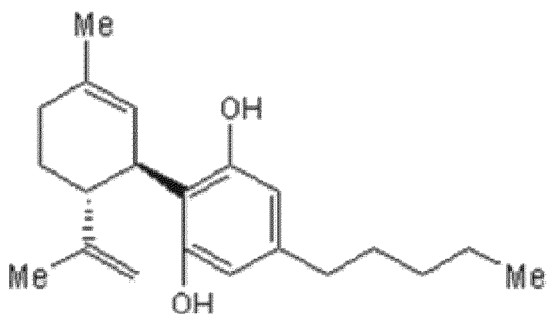

Formula I.

At present, there is no report of skin whitening methods using CBD or plant extract comprising it.

Summary

The inventors, through indepth studies and creative work, surprisingly found that cannabidiol or a cannabis extract (especially an extract of cannabis leaves) can effectively inhibit the activity of tyrosinase. The inventors further find that the cannabidiol or the cannabis extract (especially the extract of cannabis leaves) has a good skin whitening effect.

Therefore, the present invention provides a cosmetic method of skin whitening, comprising a step of providing a subject in need with an effective amount of any one of (1) to (2) below:

(1) cannabidiol or a pharmaceutically acceptable salt or ester thereof; and
(2) a plant extract containing cannabidiol, preferably, a cannabis extract containing cannabidiol, and preferably, an

industrial cannabis extract containing cannabidiol.

In a preferred embodiment the content of cannabidiol in the plant extract containing cannabidiol is 10%-99%, 20%-95%, 30%-95% or 40%-95%.

The following skin whitening product and skin whitening product set, are not part of the claimed invention and are present for illustration purposes only.

Accordingly, illustrated here is a skin whitening product, including an effective amount of any one of (1) to (2) below:

(1) cannabidiol or a pharmaceutically acceptable salt or ester thereof;
(2) a plant extract containing cannabidiol, preferably, a cannabis extract containing cannabidiol, and preferably, an industrial cannabis extract containing cannabidiol.

Preferably, the skin whitening product also includes one or more pharmaceutically or physiologically acceptable auxiliary materials.

The content of cannabidiol in the plant extract containing cannabidiol is 10%-99%, 20%-95%, 30%-95% or 40%-95%.

The skin whitening product is a composition; and preferably, the composition is cream (skin care cream), emulsion, spray, gel or patch.

Based on the calculation in weight percentage, the composition contains 0.1%-90% of active ingredient (the cannabidiol and/or the plant extract containing cannabidiol), and one or more pharmaceutically or physiologically acceptable auxiliary materials. In some embodiments of the present invention, the composition contains the active ingredient has the content of 0.1%-90%, 0.1%-50%, 0.1%-20%, 0.1%-10%, 0.1%-9%, 0.1%-8%, 0.1%-7%, 0.1%-6%, 0.1%-5%, 0.1%-4%, 0.1%-3%, 0.1%-2%, 0.1%-1%, 0.1%-0.9%, 0.1%-0.8%, 0.1%-0.7%, 0.1%-0.6%, 0.1%-0.5%, 0.1%-0.4%, 0.1%-0.3% or 0.1%-0.2%.

The term "physiologically acceptable" means that component is physiologically compatible, especially can be used in a skin topical product or can be used when in contact with skin, for example the component does not cause side effects such as irritation, etc. Especially, for example, the component may be a diluent agent, a surfactant, a thickener, an emollient and the like that can be used in the cosmetics.

By reference to a method known by those skilled in the art, the composition can be prepared to be in a proper application form or dosage form for human use, such as cream (skin care cream), emulsion, spray, gel or patch.

The skin whitening product set can include an individually packed skin whitening product.

For the subject in need, the plant extract is applied for at least one time, e.g., one time, two times, three times or more per day; the time duration for application is at least one week, at least two weeks, at least three weeks, at least four weeks or more. When the plant extract is applied more than one time per day, the time interval between two applications is at least two hours, at least four hours, at least six hours or at least eight hours. The dose for application is gradually increased from a level required for achieving a desired skin whitening effect, until the desired effect is achieved. For example, when the plant extract is applied in a cream or emulsion form, the applied amount per one square centimeter of skin is approximately at least 0.001 g, at least 0.005 g, at least 0.01 g, at least 0.02 g, at least 0.03 g, at least 0.04 g, at least 0.05 g, at least 0.06 g, at least 0.07 g, at least 0.08 g, at least 0.09 g or at least 0.1 g.

The skin whitening effect is indicated by a skin brightness value. The skin brightness value can be determined by a LAB colorimeter.

In the present invention, the cannabis extract is an extract which uses any one or more of stems, leaves, fruits, fruit shells, roots and flowers of cannabis as raw materials. Preferably, the cannabis extract is an extract of cannabis leaves. The cannabis is preferably industrial cannabis.

In the present invention, the concentrations of the ethanol or ethanol solution are based on weight percentage (wt%), unless otherwise specified.

In the present invention, the contents of cannabidiol in the cannabis extract or the extract of cannabis leaves are based on weight percentage (wt%), unless otherwise specified.

In the present invention, the term "skin care cream" refers to a product which meets the cosmetics standard QB/T 1857.

The term "subject" can refer to an animal receiving the skin whitening product of the present invention, especially a mammal, e.g., human.

Beneficial effects of the invention

The present invention achieves one or more of the following technical effects:

(1) the cannabidiol or the cannabis extract (especially the extract of cannabis leaves) can effectively inhibit activity of tyrosinase;
(2) the cannabidiol or the cannabis extract (especially the extract of cannabis leaves) can effectively inhibit synthesis of melanin;
(3) the cannabidiol or the cannabis extract (especially the extract of cannabis leaves) has a good skin whitening effect; and
(4) the cannabidiol or the cannabis extract (especially the extract of cannabis leaves) can prepare a skin whitening product, and has no toxic and side effects.

## Brief Description of the Drawings

FIG. **1** shows inhibition rates against tyrosinase by different concentrations of CBD (purity 99%).
FIG. **2** shows inhibition rates against tyrosinase by different concentrations of an extract B of cannabis leaves (containing 95% CBD).
FIG. **3** shows the inhibition rates against tyrosinase by different concentrations of extract A of cannabis leaves (containing about 50% CBD).
FIG. **4** shows the influence on relative cell survival rate by different concentrations of an extract B of cannabis leaves (containing 95% CBD).
FIG. **5** shows changes of tyrosinase activity in the cells after application of different concentrations (0.2%, 1%, 5%) of an extract B of cannabis leaves (containing 95% CBD) on B16 cell. 33 mM arbutin is used as a positive control, a cell blank is used as a negative control, a significance analysis ($p < 0.05$) is performed on experimental data, wherein ** indicates p-value < 0.01 under ANOVA TEST.
FIG. **6** shows the amount of change in melanin generated by cells after application of different concentrations of an extract B of cannabis leaves (containing 95% CBD) on B16 cell. 33 mM arbutin is used as a positive control, and a cell blank is used as a negative control, a significance analysis is performed on experimental data, wherein ** indicates p-value < 0.01 under ANOVA TEST.
FIG. **7** shows a curve of influence of an extract A of cannabis leaves (containing about 50% CBD) on skin brightness, wherein, L represents the skin brightness, the bigger its value is, the color more prefers to white color.
FIG. **8** shows a curve of influence of an extract B of cannabis leaves (containing 95% CBD) on skin brightness, wherein, L represents skin brightness, the bigger its value is, the color more prefers to white color.
FIG. **9** shows a curve of influence of CBD (purity 99%) on skin brightness, wherein, L represents the skin brightness, the bigger its value is, the color more prefers to white color.

## Detailed Description of the Embodiments

The embodiments of the present invention will be described in detail in conjunction with the Examples, but those skilled in the art would understand that, the following Examples are merely intended to illustrate the present invention, and should not be regarded as restriction on the scope of the present invention. If no specific conditions are specified in the Examples, the Examples are performed according to the conventional conditions or the conditions proposed by the manufacturer. If being not marked by manufacturers, the reagents or instruments are conventional products which are commercially available.

Preparation Example 1: preparation and detection of an extract A of cannabis leaves (containing about 50% CBD)

1. Preparation of the extract A of cannabis leaves

(1) cannabis leaves were washed, and naturally dried in the shade or oven dried at 30 °C-60 °C;
(2) pulverization was performed, and then sieving was performed through a screen of 40 meshes;
(3) coarse powder on the sieve was extracted under reflux with 10 times of 70% ethanol for two times, 1.5 hours every time, and extract solutions were combined; and
(4) concentration was performed to a relative density of 1.02 at 60 °C and under a reduced pressure to produce a product, the product was added onto a macroporous resin SP-825 (produced by Japan Mitsubishi Corporation), eluted successively with 2 fold volume (BV) of water, 4 fold volume of 45% ethanol, and 4-6 fold volume of 75% ethanol, then the column was rinsed with 3BV of 95% ethanol for regeneration; and

(5) the effluent fraction of 75% ethanol was concentrated to a relative density of 0.90-0.95 at 60 °C and under a reduced pressure, then dried for 8-12 hours under a condition of 60 °C and -0.10 MPa vacuum.

Therefore, the extract of cannabis leaves was obtained, and was named as the extract A of cannabis leaves.

2. Determination of the content of CBD in the extract A of cannabis leaves

The content of cannabidiol was determined by reference to "High Performance Liquid Chromatography (General Requirements 0512)" in the 4th Part of "Chinese Pharmacopoeia" 2015 Edition as follows:
chromatographic conditions and system suitability test: octadecyl silane chemically bonded silica was used as a filler (C18, 4.6×150 mm, 4 μm), and acetonitrile-water (volume ratio being 70 : 30) was used as a mobile phase, the detection wavelength was 210 nm, the flow rate was 1 ml/min, the column temperature was 35 °C. The number of theoretical plates number calculated based on cannabidiol peak, should not be lower than 2000.
Preparation of a reference substance solution: 0.5 ml of a cannabidiol reference substance solution (1.0 mg/ml) was measured precisely, placed into a 50 ml volumetric flask and diluted to reach the scale with 90% ethanol, the diluted solution was shaken well, and was used as the reference substance solution.
Preparation of a test sample solution: 25 mg of the sample was precisely weighed, placed into a 25 ml volumetric flask and diluted reach to the scale with 90% ethanol, the diluted solution was shaken well; 1 ml of the shaken solution was measured precisely, placed into a 100 ml volumetric flask and diluted to the scale with 90% ethanol, the diluted solution was shaken well, to obtain the test sample solution.
Determination method: 10 μl of the reference substance solution and 10 μl of the test sample solution were measured precisely and injected into a liquid chromatograph, and chromatograms were recorded. A calculation was performed based on the peak area by external standard method, to obtain the content of CBD.
The calculation formula is as follow:

$$\text{Content of cannabidiol} = \frac{\text{Asam} \times \text{Cstd} \times 25}{\text{Astd} \times \text{Wsam} \times (1-\text{Rwat\%})} \times 100\%$$

In the above formula:

Asam is an peak area of a test sample (cannabidiol);
Rwat% is water content in the test sample;
Wsam is a weighing amount of the test sample;
Astd is a peak area of a reference substance (cannabidiol); and
Cstd is nominal concentration of the reference substance (cannabidiol).

Water content determination: a Karl Fischer moisture meter and a Karl Fischer test solution were used for determination, the determined value was based on the results of parallel tests in three groups and averaged, and was recorded as Rwat%.
The determined content of cannabidiol in the extract A of cannabis leaves was 49.8 wt%.

Preparation Example 2: preparation of the extract B of cannabis leaves (containing 95% CBD)

1. Preparation of an extract B of cannabis leaves

(1) Cannabis leaves naturally dried in the shade or oven dried at 30 °C-60 °C were used, and heated for reflux for 1.5 hours with 7 times of ethanol (V/W);
(2) filtering was performed to remove residue;
(3) the filtrate was extracted with a 5% sodium hydroxide aqueous solution for two times, wherein the sodium hydroxide aqueous solution contained 20 wt% of ethanol;
(4) an extract solution was mixed with an appropriate amount of 5% sulfuric acid solution to produce a mixed solution, and the pH value of the mixed solution was 3;
(5) extraction was performed using two or one volume of ethyl acetate for two times, and then the solvent was recovered at 40 °C and -0.10 MPa vacuum to produce a product;

(6) subsequently the product is added onto an MCI column (75-150 μm, produced by Japan Mitsubishi Cooperation) for chromatography, and eluted successively with 2BV pure water, 3BV of 50% ethanol, 5BV of 75% ethanol, then the column was rinsed with 3BV 95% ethanol for regeneration; and

(7) at 60 °C and under a reduced pressure, the portion eluted with 75% ethanol was concentrated to a relative density of 0.90-0.95, then dried at 60 °C and -0.10 MPa vacuum for 8-12 hours. Therefore, the extract of cannabis leaves was produced, and was named as the extract B of cannabis leaves.

2. Determination of the content of CBD in the extract B of cannabis leaves

A determination was performed according to the method in Preparation Example 1, the determined content of cannabidiol in the extract B of cannabis leaves was 95 wt%.

Preparation Example 3: Preparation of a skin care cream (1)

The recipe is as shown in the following Table 1.

Table 1

| Phase | Reagent | Amount (mass percentage) |
|---|---|---|
| oil phase | eicosyl docosanol and eicosyl glucoside | 2 |
| | behenyl alcohol | 3.5 |
| | vaseline | 1 |
| | ethylhexyl palmitate | 6 |
| | cannabis seed oil | 1.5 |
| | extract A of cannabis leaves | 2 |
| water phase | butanediol | 5 |
| | water | 79.0 |

The extract A of cannabis leaves is prepared by Preparation Example 1; the cannabis seed oil is purchased from Yunnan Hansu Bio-technology Co., Ltd, and the COA report indicates that the cannabis seed oil does not contain CBD; and

"Eicosyl docosanol and eicosyl glucoside" is purchased from SEPPIC Corporation, and the reagent name is "MONTANOV 202" (the same below).

A preparation process is as follows:

(1) the components of the oil phase were weighed, and heated to 75 °C under a stirring condition, the temperature was maintained and sterilization was performed;

(2) the components of the water phase were weighed, and heated to 80 °C under a stirring condition, the temperature was maintained and sterilization was performed; and

(3) the oil phase was added into the water phase to produce a mixture, the mixture was homogenized for 3-5 minutes, then stirred at low speed and cooled to room temperature, to obtain a product, wherein the operation of homogenization was performed using IKA T18 digital ULTRA TURRAX homogenizer, 8000 rpm/3 min.

Therefore, a skin care cream (1) was prepared.

Preparation Example 4: preparation of a skin care cream (2)

The recipe is as shown in the following Table 2.

Table 2

| Phase | Reagent | Amount (mass percentage) |
|---|---|---|
| oil phase | eicosyl docosanol and eicosyl glucoside | 2 |
| | behenyl alcohol | 3.5 |
| | vaseline | 1 |
| | ethylhexyl Palmitate | 6 |
| | cannabis seed oil | 1.5 |
| | extract B of cannabis leaves | 1 |
| water phase | butanediol | 5 |
| | water | 80.0 |

The extract B of cannabis leaves is prepared by Preparation Example 2; the cannabis seed oil is purchased from Yunnan Hansu Bio-technology Co., Ltd, and the COA report indicates that the cannabis seed oil does not contain CBD.
The preparation process was performed by reference to the foregoing Preparation Example 3.
Therefore, the skin care cream (2) was prepared.

Preparation Example 5: preparation of a skin care cream (3)

The recipe is as shown in the following Table 3.

Table 3

| Phase | Reagent | Amount (mass percentage) |
|---|---|---|
| oil phase | eicosyl docosanolandeicosyl glucoside | 2 |
| | behenyl alcohol | 3.5 |
| | vaseline | 1 |
| | ethylhexyl palmitate | 6 |
| | cannabis seed oil | 1.5 |
| | CBD (purity 99%) | 1 |
| water phase | butanediol | 5 |
| | water | 80.0 |

The cannabidiol (purity 99%) is purchased from Yunnan Hansu Bio-technology Co., Ltd; and the cannabis seed oil is purchased from Yunnan Hansu Bio-technology Co., Ltd, and the COA report indicates that the cannabis seed oil does not contain CBD.
The preparation process was performed by reference to the above Preparation Example 3.
Therefore, a skin care cream (3) was prepared.

Comparative Preparation Example: preparation of a comparative cream

By reference to the recipe of Preparation Example 3, except that the active constituent namely the extract A of cannabis leaves was not used, the remaining constituents were identical. The preparation process was performed by reference to the above Preparation Example 3.
Therefore, the comparative cream was prepared.

Example 1: in vitro experiment

1. Experimental cell, reagent and instrument

The B16 cells are purchased from Peking Union Medical College Hospital.

The Cannabidiol (purity 99%) is purchased from Yunnan Hansu Bio-technology Co., Ltd.

The Extract A of cannabis leaves (containing about 50% CBD) is prepared by Preparation Example 1.

The extract B of cannabis leaves (containing 95% CBD) is prepared by Preparation Example 2.

DMEM high sugar medium and fetal calf serum are purchased from GIBCO Life Technologies Corporation, USA.

Levodopa, TritionX-100 and MTT are purchased from Sigma Corporation, USA.

Arbutin is purchased from Bioland Corporation.

0.05% trypsin (containing EDTA) is purchased from GIBCO Life Technologies Corporation, USA.

Tyrosinase: CAS#: 9002-10-2, is purchased from Beijing Solarbio Science & Technology Co., Ltd.

Ultraviolet specrophotometer, Japan Shimadzu Corporation, UV-2201.

WJ-80A-II type $CO_2$ constant temperature incubator, Shanghai Shengke Instrument Co., Ltd.

Enzyme linked immunosorbent detector, Thermo Fisher Scientific Instruments Co., Ltd.

2. Experimental method

(1) Determination of tyrosinase inhibition rate

B16 cells at a logarithmic phase were collected, trypsin was adopted for digestion, the digestion was terminated with a complete medium (DMEM high sugar medium + 10% fetal calf serum), counting was performed; the cell suspension was adjusted to $10\times10^4$ cells/mL, and inoculated into a 96-well culture plate, each well was added with 100 μl of cell suspension, the marginal wells were filled with sterile PBS, and cultivated in an incubator at 37 °C and with 5% $CO_2$ overnight, the medium (DMEM high sugar medium +10% fetal calf serum) was drawn out, 100 μl of the experiment sample was added into each well, and a control group (namely, the well without the sample but with only medium) was set. After action of 72 hours, 50 μl of 1% TritionX-100 solution was added for cell lysis, quickly put into an ultra-low temperature freezer at -80 °C and cryopreserved for one hour, subsequently the cryopreserved solution was thawed at room temperature, such that tyrosinase within the cells were released outside the cells, pre-warming was performed to 37 °C, then 50 μl of 1% levodopa solution was added to produce a mixture, the mixture was reacted at 37 °C for 2 hours, the absorbance value in each well was determined at the wavelength of 490 nm, for every sample concentration, more than 3 subwells were set, and averaged.

Experimental sample:

The final concentrations in each well were respectively 0.1%, 1%, 2%, 3% CBD (purity 99%);
the final concentrations in each well were respectively 0.1%, 0.5%, 1%, 2% extract B of cannabis leaves;
the final concentrations in each well were respectively 0.50%, 1%, 2%, 3% extract A of cannabis leaves.

Tyrosinase inhibition rate = 1- (mean absorbance value in each well under every concentration - mean absorbance value in the control group) × 100%.

(2) MTT experiment

B16 cells in good state at a logarithmic phase were collected, trypsin was adopted for digestion, the digestion was terminated with a complete medium, and counting was performed; the concentration of the cell suspension was adjusted to $10\times10^4$ cells/ml, inoculation was performed on a 96-well culture plate, 100 μl of cell suspension was added in each well, the marginal wells were filled with sterile PBS, and cultivation was performed in an incubator at 37 °C and with 5% $CO_2$ for 24 hours; the medium was drawn out, 10 μl of different concentrations of the samples and 90 μl of basal medium were added into each well, 100 μl of basal medium was added into the cell comparative well, and cultivation was performed in an incubator at 37 °C and with 5% $CO_2$ for 24 hours; 4 hours before the end of the cultivation, the medium containing the sample was drawn out, 10 μl of 5 mg/ml MTT solution and 90 μl of basal medium were added into each well, the cultivation was continued for 4 hours, and then the cultivation was terminated; the solutions in the wells were drawn off carefully, 100 μl of DMSO solution was added into each well, dissolution was performed at 37 °C for 10 minutes, shaking was performed, a detection was performed at the wavelength of 490 nm using ELIASA. The absorbance values in the wells were determined, for every concentration, more than 3 subwells were set, and averaged.

$$\text{Survival rate} = (\text{mean absorbance value in each well under every concentration} - \text{mean absorbance value in the control group}) \times 100\%.$$

Experimental sample:
the final concentrations of each well were respectively 5%, 1.67%, 0.56%, 0.19%, 0.06% the extract B of cannabis leaves.

(3) Determination of tyrosinase activity

B16 cells at a logarithmic phase were collected, trypsin was adopted for digestion, the digestion was terminated with a complete medium, and counting was performed; the cell suspension was adjusted to $10 \times 10^4$ cells/ml, and inoculation was performed on a 96-well culture plate, 100 μl of cell suspension was added into each well, the marginal wells were filled with sterile PBS, cultivation was performed in an incubator at 37 °C and with 5% $CO_2$ overnight, the medium were drawn out, 100 μl of experimental sample was added into each well, after action of 48 hours, 50 μl of 1% Trition X-100 solution was added for cell lysis, the lysed cells were quickly put into an ultra-low temperature freezer of -80 °C and cryopreserved for one hour, and subsequently thawed at room temperature, such that tyrosinase within the cells were released outside the cells, pre-warming was performed at 37 °C, then 50 μl of 1% levodopa solution was added into the tyrosinase to produce a mixture, the mixture was reacted at 37 °C for two hours, the absorbance values of the wells were determined at the wavelength of 490 nm, for every concentration, more than 3 subwells were set, and averaged.

Experimental sample

The final concentrations in each well were 0.2%, 1%, 5% the extract B of cannabis leaves.
The reference substances were 33 mM arbutin and cell blank (with only medium).

$$\text{Tyrosinase activity} = (\text{mean absorbance value in each well under every concentration} - \text{mean absorbance value in the control group}) \times 100\%.$$

(4) Determination of melanin content

B16 cells in good state at a logarithmic phase were collected, trypsin was adopted for digestion, the digestion was terminated with a complete medium, and counting was performed; the prepared cell suspension was adjusted to $5 \times 10^4$ cells/ml and added into a 6-well culture plate, 2 ml of cell suspension was added into each well, cultivation was performed in an incubator at 37 °C and with 5% $CO_2$ overnight; the medium was drawn out, 2 ml of cannabis extracts with different concentrations were added into each well, after action of 48 hours, the medium was drawn out and abandoned, trypsin was adopted for digestion, and the product was collected into a centrifugal tube, centrifugation was performed to obtain a cell precipitate, the cell precipitate was washed with PBS for one time, a 1M NaOH (containing 10% DMSO) solution was added for cell lysis, and the lysed cells were put in a 80 °C water bath kettle for water bath for 30 minutes; uniform shaking and mixing were performed, and the processed cells were drawn into to a 96-well plate; and the absorbance value was determined at the wavelength of 245 nm.

$$\text{Relative melanin content} = (\text{mean absorbance value in each well under every concentration} - \text{mean absorbance value in the control group}) \times 100\%.$$

3. Experimental results

(1) The inhibitory effects of the cannabis extract against tyrosinase are as shown in FIG. **1**, FIG. **2** and FIG. **3**.
The results show that, CBD (99% purity), the extract B of cannabis leaves (containing 95% CBD) and the extract A of cannabis leaves (containing 49.8% CBD) have an effective inhibitory effect against tyrosinase, and for every drug, with increase of the amount, the inhibition rate against tyrosinase is increased.

(2) The results of B16-MTT experiment are as shown in FIG. **4**.

The results show that, within a certain range, the survival rate of B16 cells is reduced with increase of the concentration of the extract of cannabis leaves, and there is a certain concentration dependency. After action of 48 hours by the extract B of cannabis leaves (containing 95% CBD) with a concentration of 5%, the cell viability is lowest, being 90.88%; and under other concentrations, the cell survival rates are 90.88% or above. By comparison under different concentrations, there was no significant difference and no cytotoxicity.

(3) The results of changes in tyrosinase activity are as shown in FIG. **5**.

The results show that, the extract B of cannabis leaves with concentrations of 0.2%, 1%, and 5% can significantly reduce the tyrosinase activity within the cells; with increase of the concentration of the extract B of cannabis leaves, tyrosinase activity was reduced, wherein the extract B of cannabis leaves with a concentration of 5% had a best inhibitory effect against tyrosinase, and reduce the tyrosinase activity to 73.13%.

(4) The results of changes in the melanin content are as shown in FIG. **6**.

The results show that different concentrations of the extract B of cannabis leaves (containing 95% CBD) can significantly reduce the amount of melanin synthesized by the cells, even can reduce the melanin content within the B16 cells to previous 73.79%, and reduce to 79.24% in the positive control; and with increase in concentration of the extract B of cannabis leaves (95% CBD), the melanin content becomes lower. Influence of different concentrations of the extract of cannabis leaves on melanin synthesis has no significant difference compared with arbutin.

Example 2: cytotoxicity test (MTT)

1. Experimental samples

The extract A of cannabis leaves (containing about 50% CBD) is prepared by Preparation Example 1.
The extract B of cannabis leaves (containing 95% CBD) is prepared by Preparation Example 2.
Cannabidiol (purity 99%) is purchased from Yunnan Hansu Biotechnology Co., Ltd.
Cannabis seed oil is purchased from Yunnan Hansu Bio-technology Co., Ltd, and COA report indicates that the cannabis seed oil does not contain CBD.
Human immortalized epidermal cell is purchased from Peking Union Medical College Hospital.
MTT is purchased from Sigma Corporation, USA.
DMEM high sugar medium and fetal calf serum are purchased from GIBCO Life Technologies Corporation, USA.
PBS is purchased from Corning Corporation, USA.
WJ-80A-II type $CO_2$ constant temperature incubator is purchased from Shanghai Shengke Instrument Co., Ltd.
Enzyme linked immunosorbent detector is purchased from Thermo Fisher Scientific Instruments Co., Ltd.

2. Experimental method

Human immortalized epidermal cells at a logarithmic phase were collected, the concentration of the cell suspension was adjusted, the cell suspension was added to a 96-well plate at 100 μl per well, incubation was performed at 37 °C and with 5% $CO_2$ to reach a cell density of 5000 cells/well. The solution was changed and the experimental samples at different concentration gradients were added (as the following Table 4), a culture solution containing no experiment sample was used as the control. Incubation was performed with 5% $CO_2$ and at 37 °C for 24 hours, 20 μl of MTT solution (5 mg/ml, i.e. 0.5%MTT) was added into each well, the cultivation was continued for four hours. The drug reacted with MTT sufficiently, firstly centrifugation was performed, then the culture solution was abandoned, and rinsing was performed with PBS carefully for 2-3 times, then a culture solution containing MTT was added. The cultivation was terminated, the culture solution in the well were drawn off carefully. 150 μl of dimethyl sulfoxide was added into each well, and placed onto a shaker and shaken at a low speed for 10 minutes, such that the crystal substances were sufficiently dissolved. The absorbance values of each well were measured in an enzyme linked immunosorbent detector at OD value of 490 nm.

$$\text{Cell survival rate} = (\text{determined well OD value - blank control OD value})/(\text{OD value in the cell comparative group - OD value in the blank control}) \times 100\%.$$

Table 4: Recipe of the sample used in cytotoxicity test of human immortalized epidermal cells (the content of each component is in mass percentage)

| | Blank | Sample A | Sample B | Sample C | Sample D | Sample E |
|---|---|---|---|---|---|---|
| deionized water | 99 | 98 | 88 | 86 | 85 | 86 |
| cocamidopropyl betaine | 1 | 1 | 1 | 1 | 1 | 1 |
| polyacrylate cross-linked polymer-6 | | 1 | 1 | 1 | 1 | 1 |
| cannabis seed oil | | | 10 | 10 | 10 | 10 |
| CBD (99% purity) | | | | | | 2 |
| extract B of cannabis leaves | | | | 2 | | |
| extract A of cannabis leaves | | | | | 3 | |

3. The experimental results are as shown in the following Table 5.

Table 5

| Name of samples | Final concentrations of the samples in each well | Cell survival rate |
|---|---|---|
| Blank | 1.00% | 80.78% |
| Blank | 0.50% | 85.40% |
| Blank | 0.20% | 86.80% |
| Sample A | 1.00% | 72.75% |
| Sample A | 0.50% | 78.82% |
| Sample A | 0.20% | 86.98% |
| Sample B | 1.00% | 64.06% |
| Sample B | 0.50% | 73.90% |
| Sample B | 0.20% | 74.64% |
| Sample C | 1.00% | 74.88% |
| Sample C | 0.50% | 78.38% |
| Sample C | 0.20% | 83.20% |
| Sample D | 1.00% | 75.93% |
| Sample D | 0.50% | 73.49% |
| Sample D | 0.20% | 87.95% |
| Sample E | 1.00% | 75.12% |
| Sample E | 0.50% | 79.48% |
| Sample E | 0.20% | 84.56% |

The results show that, the cannabis seed oil added has a certain toxicity on the cells. CBD (99% purity), the extract B of cannabis leaves (containing 95% CBD) and the extract A of cannabis leaves (containing about 50% CBD) can promote cell proliferation to a certain extent to eliminate the influence of the cannabis seed oil.

Example 3: skin whitening experiment

1. Experimental samples, instruments and experiment objects

Experiment samples: the skin care creams (1) to (3) prepared by Preparation Examples 3 to 5. The comparative cream prepared by the Comparative Preparation Example.

Instrument: LAB colorimeter (MPA9, produced by CK Electronics Corporation, Germany).

Objects: testers for every experiment sample are 25 persons, including 10 men and 15 women, their ages are all between 30 to 55 years old.

2. Experimental method (using one experimental sample as the example)

(1) Before smearing of the sample, the subject firstly washed the experiment site, and smeared the sample after airing. In a symmetrical manner, areas with dimension of 4×4 cm on insides of left and right arms of the subject were respectively identified as a test area and a comparative area.

(2) The subject evenly smeared a proper amount (about 0.5 g) of the cream onto the test area, meanwhile the subject evenly smeared the comparative cream (as blank matrix) onto the control area. Every day the subject smeared one time in the morning and one time in the evening, the time interval between the two smearing was about 8-10 hours. During the experiment, the subject should not smear any cosmetics at the experiment area.

(3) After continuous use of the sample, the subject tested the change in skin color using Lab colorimeter at the same time every week, and an average value was used. The experiment lasted for 4 weeks.

(4) Statistics were conducted on the determined values of each time: skin brightness values (LAB value) in the control group (comparative areas on right arms of 25 persons) and in the experimental group (test areas on left arms of 25 persons).

3. The experimental results are as shown in Table 6-8 and FIG. 7-9.

Table 6: skin care cream (1), using the extract A of cannabis leaves

| | Mean Value of Skin Brightness | |
|---|---|---|
| Time (week) | Control Group | Experimental Group |
| 0 | 62.3 | 62.4 |
| 1 | 62.6 | 53.1 |
| 2 | 62.9 | 54.3 |
| 3 | 63.7 | 56.5 |
| 4 | 64.1 | 58 |

Table 7: skin care cream (2), using the extract B of cannabis leaves

| | Mean Value of Skin Brightness | |
|---|---|---|
| Time (week) | Control Group | Experimental Group |
| 0 | 62.3 | 52.3 |
| 1 | 62.5 | 53.1 |
| 2 | 63.6 | 54.7 |
| 3 | 63.8 | 56.3 |
| 4 | 64.3 | 57.2 |

Table 8: skin care cream (3), using CBD with 99% purity

| | Mean Value of Skin Brightness | |
|---|---|---|
| Time (week) | Control Group | Experimental Group |
| 0 | 62 | 62.1 |
| 1 | 62.1 | 52.6 |
| 2 | 63.4 | 54.3 |
| 3 | 63.7 | 56.2 |
| 4 | 64.3 | 57.1 |

Before use of the samples, there is no significant difference between the skin brightness of the three experimental groups and the skin brightness of the control group. The results show that, after use of the samples, at week 1, the changes in the skin brightness value are not significant, but at week 2-4, the skin brightness values in the three experimental groups all increase significantly.

## Claims

1. A cosmetic method of skin whitening, comprising a step of providing a subject in need with an effective amount of any one of (1) to (2) below:

   (1) cannabidiol or a pharmaceutically acceptable salt or ester thereof; and
   (2) a plant extract containing cannabidiol, preferably, a cannabis extract containing cannabidiol, and preferably, an industrial cannabis extract containing cannabidiol.

2. The method according to claim 1, wherein the content of cannabidiol in the plant extract containing cannabidiol is 10%-99%, 20%-95%, 30%-95% or 40%-95%.

## Patentansprüche

1. Ein kosmetisches Verfahren zur Hautbleichung, das einen Schritt des Bereitstellens einer effektiven Menge von einem von (1) bis (2) unten für ein Individuum mit Bedarf beinhaltet:

   (1) Cannabidiol oder einem pharmazeutisch akzeptablen Salz oder einem pharmazeutisch akzeptablen Ester davon; und
   (2) einem Pflanzenextrakt, das Cannabidiol enthält, vorzugsweise einem Cannabisextrakt, das Cannabidiol enthält, und vorzugsweise einem industriellen Cannabisextrakt, das Cannabidiol enthält.

2. Verfahren gemäß Anspruch 1, wobei der Gehalt an Cannabidiol in dem Pflanzenextrakt, das Cannabidiol enthält, 10 %-99 %, 20 %-95 %, 30 %-95 % oder 40 %-95 % beträgt.

## Revendications

1. Un procédé cosmétique de blanchiment de la peau, comprenant une étape de fourniture à un sujet en ayant besoin d'une quantité efficace de l'un quelconque parmi (1) et (2) ci-dessous :

   (1) du cannabidiol ou un sel ou ester pharmaceutiquement acceptables de celui-ci ; et
   (2) un extrait de plante contenant du cannabidiol, de préférence, un extrait de cannabis contenant du cannabidiol, et de préférence, un extrait de cannabis industriel contenant du cannabidiol.

2. Le procédé selon la revendication 1, dans lequel la teneur en cannabidiol dans l'extrait de plante contenant du cannabidiol est de 10 % à 99 %, de 20 % à 95 %, de 30 % à 95 % ou de 40 % à 95 %.

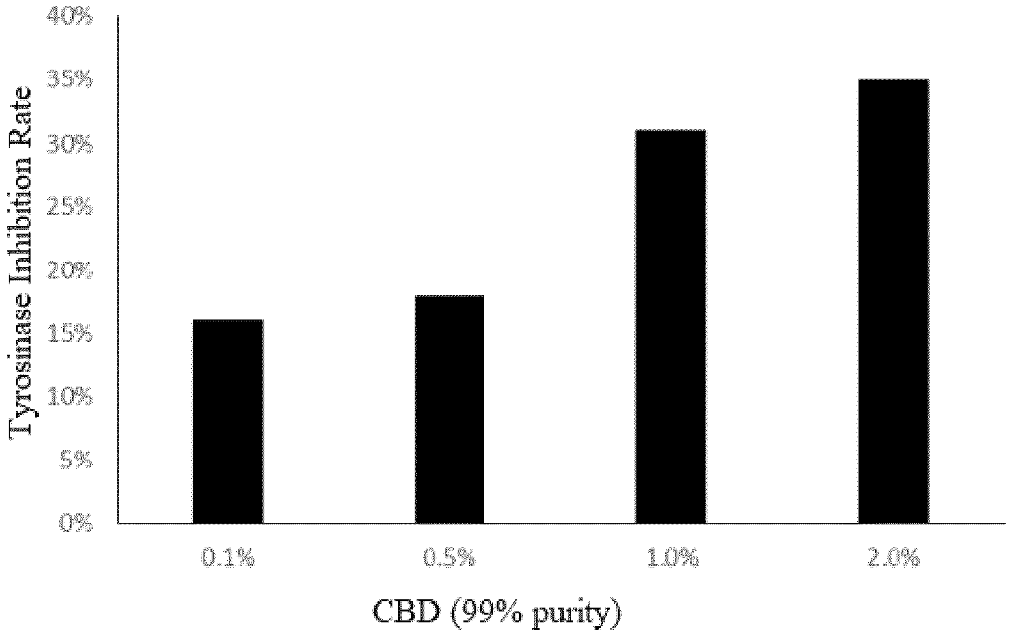
Tyrosinase Inhibition Rate
40%
35%
30%
25%
20%
15%
10%
5%
0%
0.1%
0.5%
1.0%
2.0%
CBD (99% purity)

Fig. 1

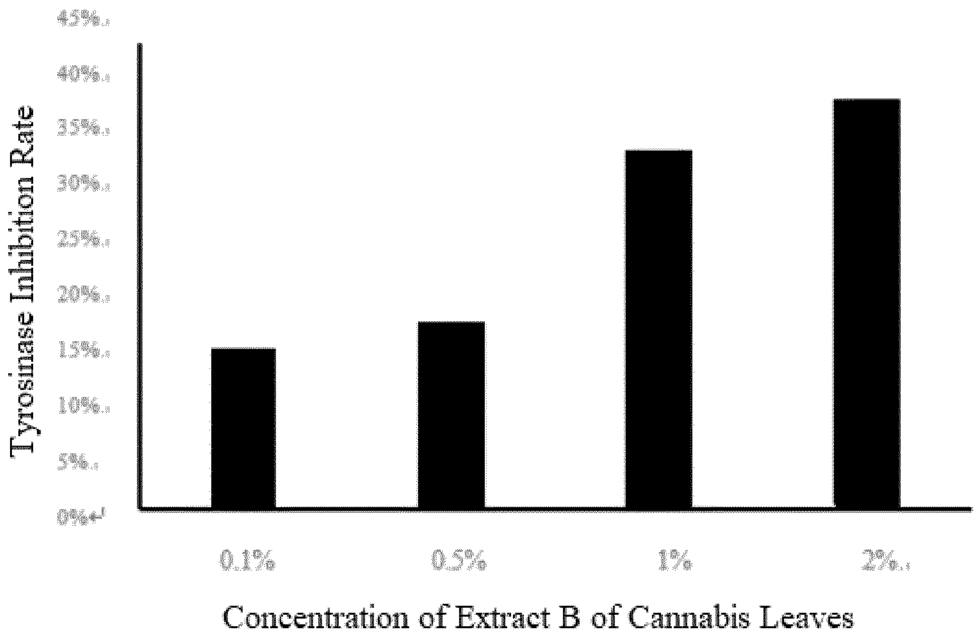
Tyrosinase Inhibition Rate
45%
40%
35%
30%
25%
20%
15%
10%
5%
0%
0.1%
0.5%
1%
2%
Concentration of Extract B of Cannabis Leaves

Fig. 2

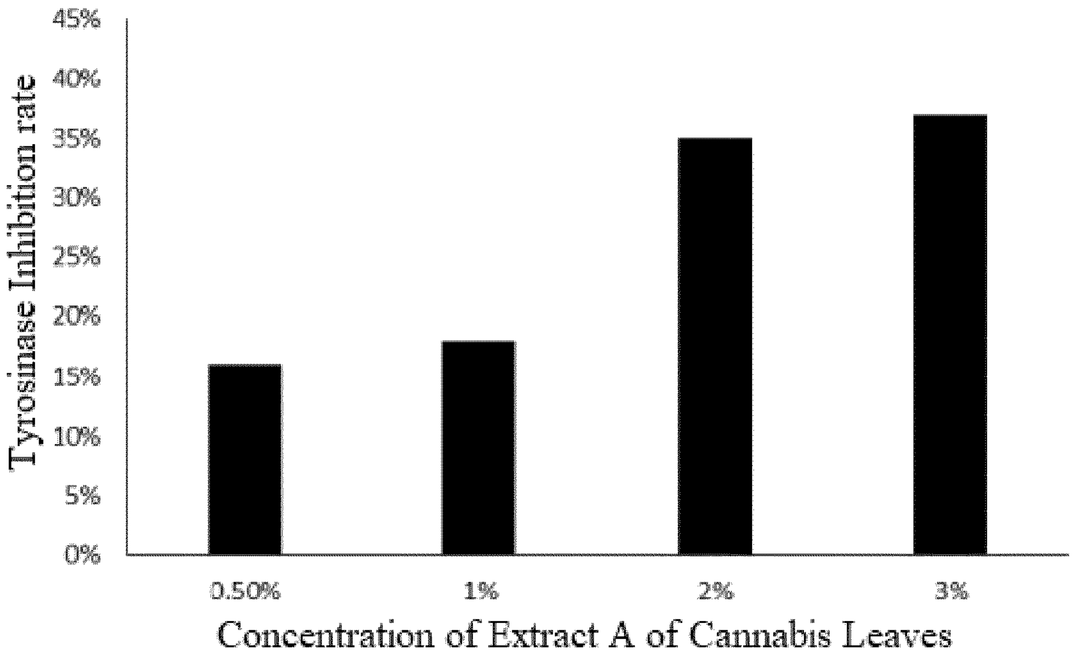
Tyrosinase Inhibition rate
45%
40%
35%
30%
25%
20%
15%
10%
5%
0%
0.50%
1%
2%
3%
Concentration of Extract A of Cannabis Leaves

Fig. 3

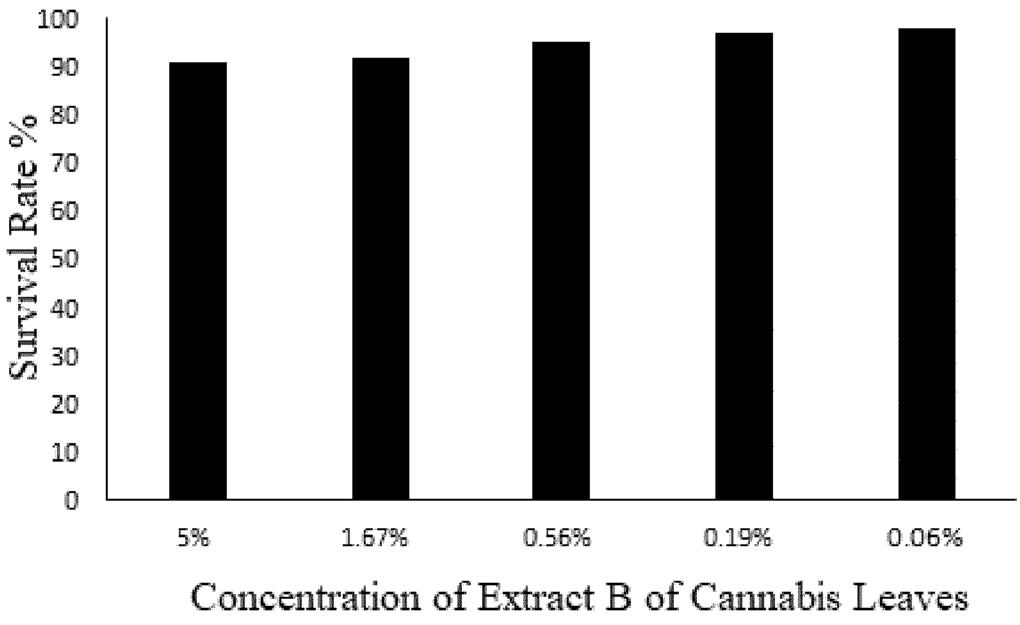
Survival Rate %
100
90
80
70
60
50
40
30
20
10
0
5%
1.67%
0.56%
0.19%
0.06%
Concentration of Extract B of Cannabis Leaves

Fig. 4

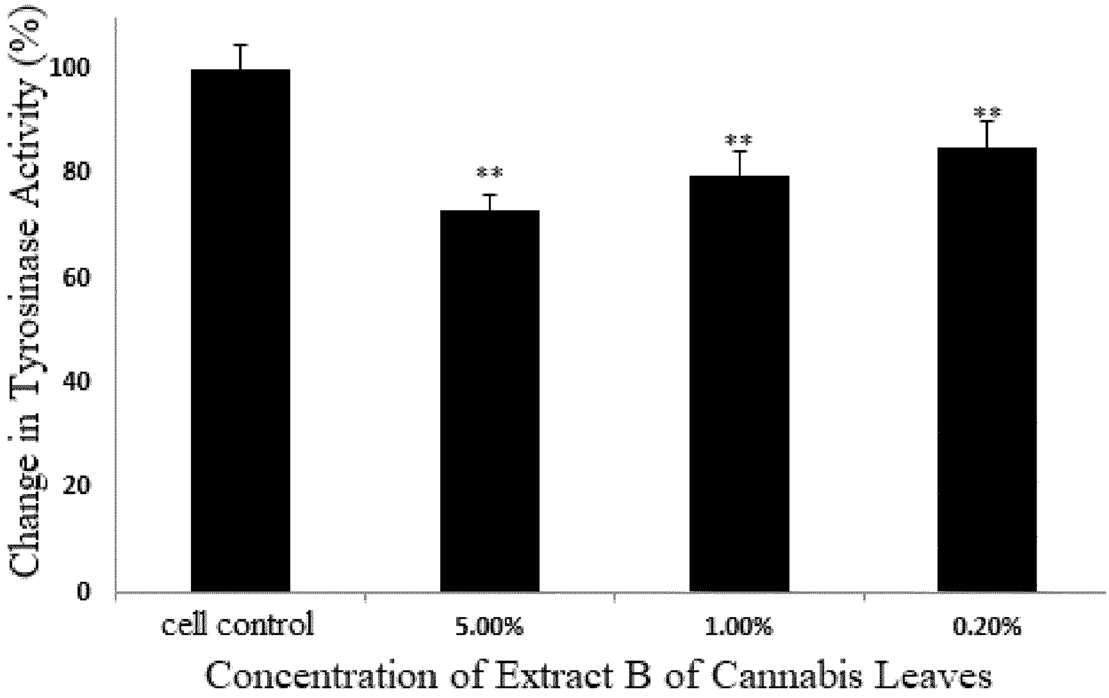
Change in Tyrosinase Activity (%)
100
80
60
40
20
0
**
**
**
cell control
5.00%
1.00%
0.20%
Concentration of Extract B of Cannabis Leaves

Fig. 5

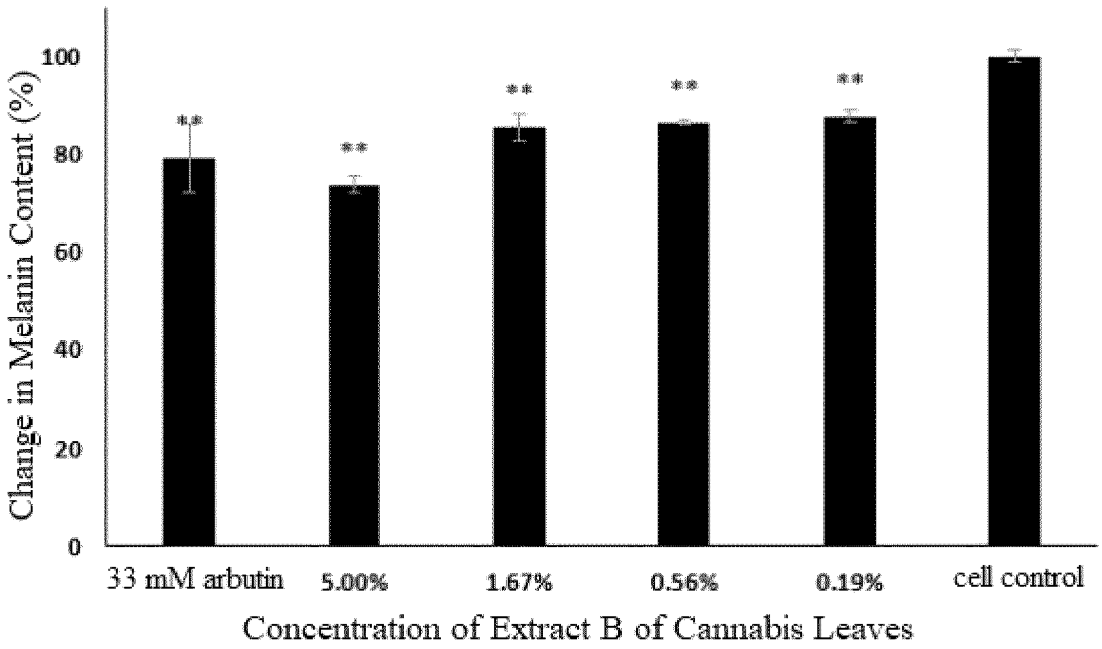
Change in Melanin Content (%)
100
80
60
40
20
0
**
**
**
**
**
33 mM arbutin
5.00%
1.67%
0.56%
0.19%
cell control
Concentration of Extract B of Cannabis Leaves

Fig.6

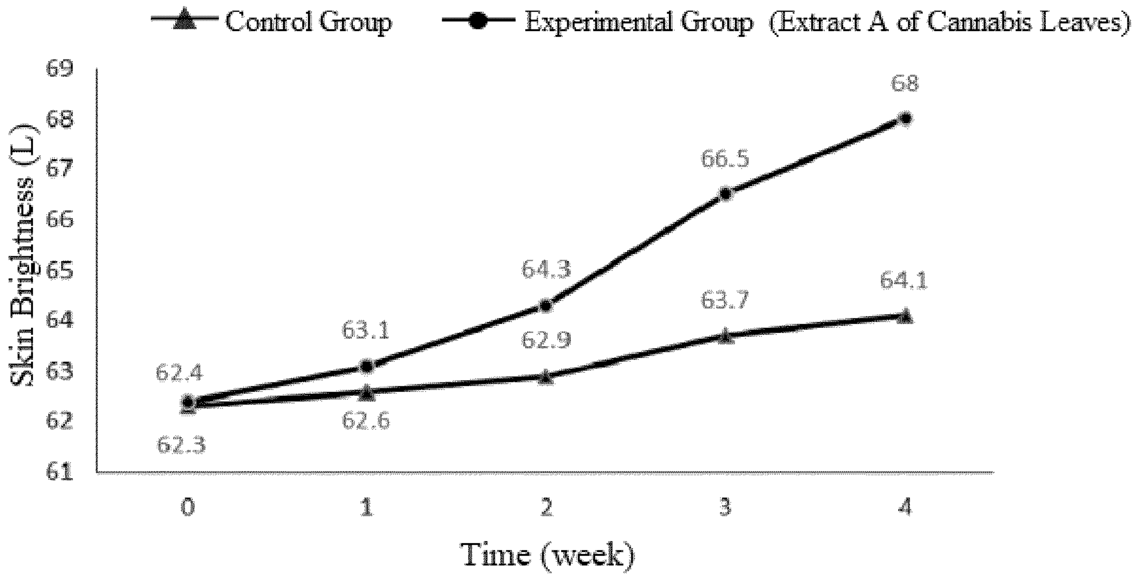

Control Group
Experimental Group (Extract A of Cannabis Leaves)
Skin Brightness (L)
69
68
67
66
65
64
63
62
61
68
66.5
64.3
64.1
63.7
63.1
62.9
62.4
62.6
62.3
0
1
2
3
4
Time (week)


Fig. 7

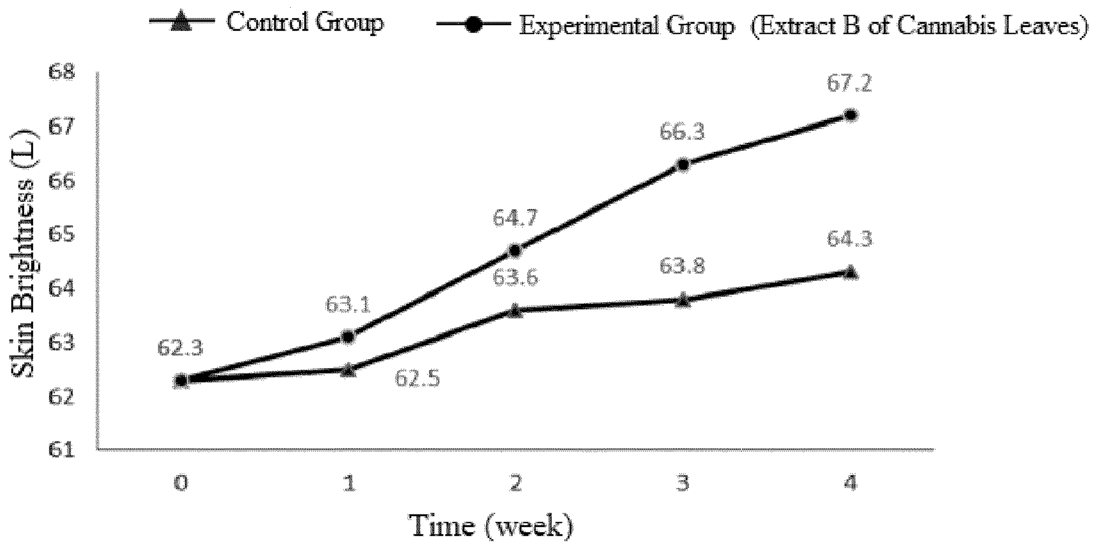

Control Group
Experimental Group (Extract B of Cannabis Leaves)
Skin Brightness (L)
68
67
66
65
64
63
62
61
67.2
66.3
64.7
64.3
63.8
63.6
63.1
62.3
62.5
0
1
2
3
4
Time (week)


Fig. 8

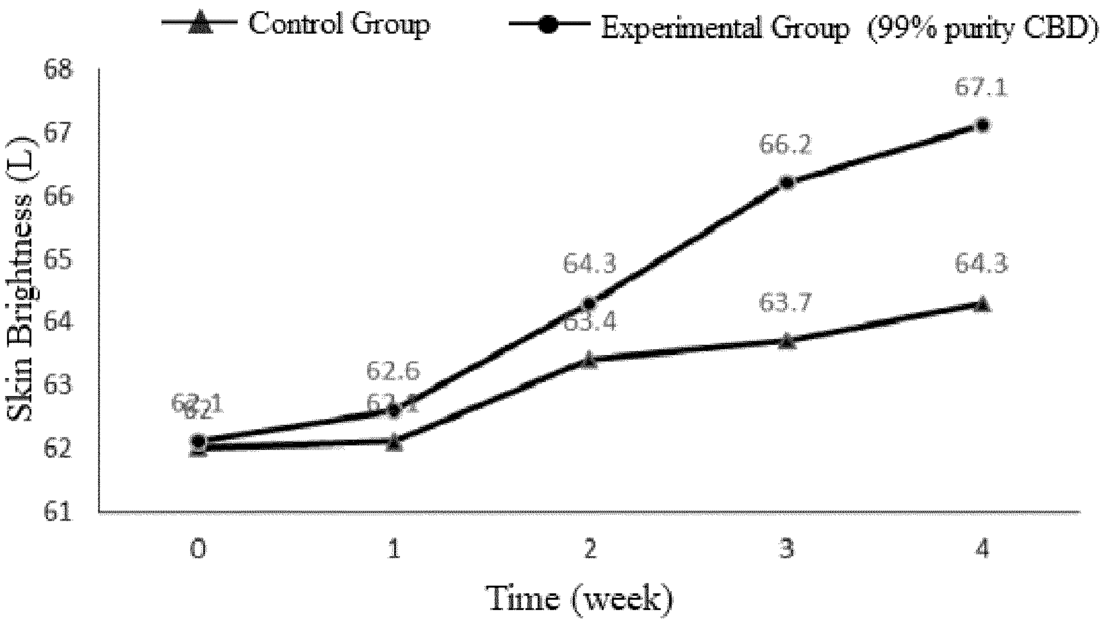
Control Group
Experimental Group (99% purity CBD)
68
67
66
65
64
63
62
61
Skin Brightness (L)
67.1
66.2
64.3
64.3
63.7
63.4
62.6
0
1
2
3
4
Time (week)

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107308440 A
- US 2016000843 A
- US 20160374911 A1


### Non-patent literature cited in the description


- **HWANG YOUNG SUN et al.** Cannabidiol upregulates melanogenesis through CB1 dependent pathway by activating p38 MAPK and p42/44 MAPK. *CHEMICO-BIOLOGICAL INTERACTIONS,* 2017, vol. 273, 107-114